# FASCICULE DE BREVET EUROPEEN

(11) **EP 3 001 915 B1**
(45) Date de publication et mention de la délivrance du brevet: **17.10.2018**
(21) Numéro de dépôt: 15290222.7
(22) Date de dépôt: 26.08.2015
(51) Int. Cl.: A61K 36/02, A23L 27/12, A23L 33/10, A23L 33/105, A23L 33/115, A23L 17/60

(54) **PROCÉDÉ DE FABRICATION D'UN COMPLÉMENT ALIMENTAIRE À BASE D'HUILES ESSENTIELLES EN POUDRE**
HERSTELLUNGSVERFAHREN EINES NAHRUNGSERGÄNZUNGSMITTELS AUF DER BASIS VON ÄTHERISCHEN ÖLEN IN PULVERFORM
METHOD FOR PRODUCING A FOOD SUPPLEMENT MADE OF ESSENTIAL OILS IN POWDER FORM

(30) Priorité: 12.09.2014 FR 1402039
(43) Date de publication de la demande: 06.04.2016
(73) Titulaire: Obriot, Bruno, 22970 Ploumagoar (FR)
(72) Inventeur: Obriot, Bruno, 22970 Ploumagoar (FR)
(74) Mandataire: Flavenot, Bernard

(56) Documents cités:
- EP-A1- 1 938 810
- EP-A2- 0 110 638
- EP-A2- 0 240 067
- WO-A1-2006/000652
- WO-A2-2004/028550
- DE-A1-102006 056 454
- FR-A1- 2 572 935
- FR-A1- 2 941 846
- US-A- 4 973 485
- US-A1- 2010 303 990

## Description

La présente invention concerne les procédés de fabrication de compléments alimentaires à base d'huiles essentielles en poudre.

Les produits réalisés à base d'huiles essentielles constituent de parfaits compléments alimentaires qui, sans être des médicaments, contribuent incontestablement au bien-être des êtres vivants, humains ou analogues.

Le problème qui se pose est celui de leur absorption par les êtres vivants. En effet, la forme liquide s'avère peu pratique. Aussi, il a été tenté de réaliser ces compléments alimentaires à base d'huiles essentielles sous forme solide. Un tel procédé est par exemple décrit dans les documents FR2572935, WO/2006000652 et/ou WO2004/028550, éventuellement EP1938810, US2010/303990, DE102006056454 et FR2941846.

Sont également connus des procédés de fabrication de compléments alimentaires comme ceux qui sont évoqués dans les documents FR 2 572 935, WO 2006/000652, WO 2004/028550, EP 1 938 810, US 2010/303990, DE 10 2006 056454 et FR 2 941 846.

Ces documents ne décrivent pas des procédés faciles à mettre en oeuvre avec des moyens couramment disponibles sur le marché et, surtout, qui permettent d'obtenir des produits satisfaisants pour constituer des compléments alimentaires à base d'huiles essentielles en poudre pour le bien des consommateurs.

En fait, tous les résultats obtenus jusqu'à ce jour ne donnent pas réellement satisfaction, et les procédés pour réaliser ces solides sont relativement complexes et donc onéreux.

Aussi, la présente invention a-t-elle pour but de mettre en oeuvre un procédé de fabrication de compléments alimentaires à base d'huiles essentielles en poudre qui pallie en grande partie les inconvénients des procédés de l'art antérieur.

Plus précisément, la présente invention a pour objet un procédé de fabrication d'un complément alimentaire à base d'au moins une huile essentielle en poudre, ayant les caractéristiques listées dans la revendication 1 annexée.

D'autres caractéristiques et avantages de la présente invention apparaîtront au cours de la description suivante donnée en regard d'un exemple de mise en oeuvre du procédé selon l'invention pour la fabrication d'un complément alimentaire à base d'huiles essentielles en poudre, cet exemple de mise en oeuvre étant donné ci-après à titre illustratif mais nullement limitatif.

Les huiles essentielles, parfois désignées sous le terme "d'essences végétales" sont bien connues. Elles sont utilisées dans de nombreux domaines d'application, notamment pour le bien-être des êtres vivants, aussi bien l'être humain que les animaux. Elles sont constituées d'un liquide concentré et hydrophobe des composés aromatiques volatils d'une plante. Ces composés sont obtenus par extraction mécanique, entraînement à la vapeur d'eau ou distillation à sec. Il est aussi possible d'extraire ces composés végétaux avec des solvants non aqueux volatils, comme des hexanes, ou des éthers. On peut aussi citer l'extraction au CO₂ supercritique.

La présente invention concerne un procédé pour la fabrication d'un complément alimentaire en poudre à base d'au moins une huile essentielle en poudre.

Ce procédé consiste tout d'abord à pré-centrifuger une certaine quantité d'algue en poudre, à une vitesse de centrifugation V1 en tours par minute (tr/min).

De préférence cette algue est du lithothamne qui est une algue relativement facile à mettre en poudre car elle est très calcaire. Le lithothamne est une algue que l'on trouve dans les fonds marins. Il se présente sous forme de petits bourgeons qui ressemblent à du corail et appartient d'ailleurs à la famille des Corallinacéees. Il possède donc la capacité de synthétiser les bienfaits de la mer et constitue ainsi une sorte d'eau de mer à l'état solide.

L'intérêt majeur de cette algue est le fait qu'elle soit spécialement riche en minéraux et en oligoéléments. Le lithothamne est en effet particulièrement riche en minéraux tels que le calcium (45%) et le magnésium (10%). Il contient également de nombreux oligoéléments tels que l'iode, le soufre, le fer, le manganèse, le zinc, le sélénium, le bore, le cuivre, le cobalt, le sodium ou encore la silice.

Il peut de ce fait entrer dans la fabrication d'un complément alimentaire idéal contenant à lui seul 19 des 32 oligoéléments essentiels à la bonne santé du corps humain. Sa haute teneur en calcium permet en particulier de conserver un bon capital osseux ; sa haute teneur en fer permet de lutter efficacement contre l'anémie. Il se révèle également être une source importante de Vitamine C.

Cette algue peut donc, d'une part être utilisée de manière régulière comme complément alimentaire pour récupérer les différentes carences qui peuvent apparaître à cause d'une mauvaise alimentation ou hygiène de vie, et d'autre part être utilisée pour agir sur toutes sortes de problématiques au niveau du corps humain.

Le procédé consiste ensuite à porter la vitesse de centrifugation à une valeur V2 en tours par minute (tr/min) supérieure à V1 tout en mélangeant, avec la certaine quantité d'algue pré-centrifugée, une certaine quantité d'huile(s) essentielle(s) pulvérisée portée à une température déterminée, pour obtenir un mélange algue-huile essentielle dans un premier état qui est en fait semi pâteux.

Une telle huile essentielle est choisie très avantageusement parmi les huiles essentielles suivantes : lavande vraie, romarin à verbénone, anis vert, arbre à thé, citron, cajeput, eucalyptus citronné, pin sylvestre, laurier, un mélange d'au moins deux de ces huiles essentielles.

La centrifugation du mélange algue-huile essentielle dans ledit premier état est maintenue à la vitesse V2 pendant une durée de temps T1, jusqu'à obtenir un mélange algue-huile essentielle dans un deuxième état qui est toujours pâteux, mais cependant un peu plus épais.

Le procédé consiste alors à soumettre le mélange algue-huile essentielle dans ledit deuxième état à une pression déterminée pendant une durée de temps T2 sous atmosphère de gaz neutre, de préférence de l'air, pour obtenir un mélange algue-huile essentielle dans un troisième état qui se présente comme une "poudre humide".

A la fin de cette durée de temps T2, le mélange algue-huile essentielle dans ledit troisième état est ramené à la pression atmosphérique et à la température ambiante, pour obtenir un mélange algue-huile essentielle dans un quatrième état qui se présente toujours sous la forme d'une "poudre humide".

Le mélange algue-huile essentielle dans ledit quatrième état est alors soumis à une autre centrifugation à la vitesse V3 en tours par minute (tr/min), tout en ajoutant une certaine quantité d'extrait sec de plantes et de silice colloïdale, pendant une durée de temps T3, pour obtenir un mélange final dans un cinquième état.

De façon préférentielle, l'extrait sec de plantes est choisi parmi les extraits secs des plantes suivantes : mélisse, cône de houblon, origan vert, canneberge, un mélange d'au moins deux de ces plantes.

On laisse ensuite reposer le mélange final dans ledit cinquième état, pendant une durée de temps T4, pour obtenir une poudre apte à constituer le complément alimentaire recherché, à base d'huile(s) essentielle(s) en poudre.

Dans une mise en oeuvre préférentielle, les vitesses de centrifugation V1, V2 et V3 sont sensiblement égales respectivement à 3000, 4500 et 5000 tr/min, les durées de temps T1, T2, T3 et T4 sont sensiblement égales respectivement à une heure, quatre heures, quinze minutes et vingt-quatre heures, la valeur de la température déterminée est sensiblement égale à 32°C, et la valeur de la pression déterminée est sensiblement égale à trois bars.

De façon avantageuse, pour obtenir une poudre ayant des qualités optimales, les proportions en poids des quantités définies ci-avant, respectivement d'algue, d'huile essentielle, de silice colloïdale et d'extraits secs de plantes sont respectivement sensiblement égales à 65%, 15%, 1% et 19%.

Il est par ailleurs préférable que, pour obtenir un résultat très acceptable, l'extrait sec de plantes soit de l'ordre de 5/1, c'est-à-dire qu'une unité de poids d'extrait sec équivaut en moyenne à cinq unités de poudre de plante.

Pour assurer une bonne conservation du mélange en poudre final ainsi obtenu et faciliter sa distribution, il est ensuite possible, et avantageux, de le conditionner en gélules.

La présenté invention a aussi pour objet un dispositif pour mettre en oeuvre le procédé décrit ci-dessus. Ce dispositif comporte au moins, en combinaison, une centrifugeuse commandable en vitesse de rotation V (tr/min), un atomiseur pour pulvériser l'huile essentielle, une cuve chauffante, un caisson hyperbare.

La présente invention a aussi pour objet le complément alimentaire à base d'au moins une huile essentielle en poudre obtenu par le procédé décrit ci-dessus.

## Revendications

1. Procédé de fabrication d'un complément alimentaire à base d'au moins une huile essentielle en poudre, **caractérisé par le fait qu'**il consiste :
• à pré-centrifuger une certaine quantité d'algue à une vitesse de centrifugation V1 en tours par minute (tr/min),
• à porter la vitesse de centrifugation à une valeur V2 en tours par minute (tr/min) supérieure à V1 tout en mélangeant, avec ladite certaine quantité d'algue pré-centrifugée, une certaine quantité d'huile essentielle pulvérisée portée à une température déterminée, pour obtenir un mélange algue-huile essentielle dans un premier état,
• à maintenir, à vitesse V2, la centrifugation du mélange algue-huile essentielle dans ledit premier état pendant une durée de temps T1, pour obtenir un mélange algue-huile essentielle dans un deuxième état,
• à soumettre le mélange algue-huile essentielle dans ledit deuxième état à une pression déterminée pendant une durée de temps T2 sous atmosphère d'un gaz donné pour obtenir un mélange algue-huile essentielle dans un troisième état,
• à ramener, à la fin de cette durée de temps T2, le mélange algue-huile essentielle dans un troisième état, à la pression atmosphérique et à la température ambiante, pour obtenir un mélange algue-huile essentielle dans un quatrième état,
• à soumettre le mélange algue-huile essentielle dans ledit quatrième état, à une autre centrifugation à la vitesse V3 en tours par minute (tr/min) tout en ajoutant une certaine quantité d'extrait sec de plantes et de silice colloïdale, pendant une durée de temps T3, pour obtenir un mélange final dans un cinquième état, et
• à laisser reposer ledit mélange final dans ledit cinquième état pendant une durée de temps T4, pour obtenir une poudre apte à constituer ledit complément alimentaire à base d'huile essentielle en poudre.

2. Procédé selon la revendication 1, **caractérisé par le fait que** :
• les vitesses de centrifugation V1, V2 et V3 sont sensiblement égales respectivement à 3000, 4500 et 5000 tr/min,
• les durées de temps T1, T2, T3 et T4 sont sensiblement égales respectivement à une heure, quatre heures, quinze minutes et vingt-quatre heures,
• la valeur de la température déterminée est sensiblement égale à 32°C, et
• la valeur de la pression déterminée est sensiblement égale à trois bars.

3. Procédé selon l'une des revendications 1 et 2, **caractérisé par le fait que** les proportions en poids des dites certaines quantités d'algue, d'huile essentielle, de silice colloïdale et d'extraits secs de plantes sont respectivement sensiblement égales à 65%, 15%, 1% et 19%.

4. Procédé selon l'une des revendications précédentes, **caractérisé par le fait que** ladite algue est du lithothamne.

5. Procédé selon l'une des revendications précédentes, **caractérisé par le fait que** l'huile essentielle est choisie parmi les huiles essentielles suivantes : lavande vraie, romarin à verbénone, anis vert, arbre à thé, citron, cajeput, eucalyptus citronné, pin sylvestre, laurier, un mélange d'au moins deux de ces huiles essentielles.

6. Procédé selon l'une des revendications précédentes, **caractérisé par le fait que** l'extrait sec de plantes est choisi parmi les extraits secs des plantes suivantes : mélisse, cône de houblon, origan vert, canneberge, un mélange d'au moins deux de ces plantes.

7. Procédé selon l'une des revendications précédentes, **caractérisé par le fait que** l'extrait sec de plantes est de l'ordre de 5/1, c'est-à-dire qu'une unité de poids d'extrait sec équivaut en moyenne à cinq unités de poudre de plante.

8. Procédé selon l'une des revendications précédentes, **caractérisé par le fait que** ledit mélange final est mis en gélule.

## Patentansprüche

1. Verfahren zum Herstellen eines Nahrungsergänzungsmittels auf der Basis mindestens eines ätherischen Öls in Pulverform, **gekennzeichnet durch**:
- Vorzentrifugieren einer bestimmten Algenmenge mit einer Zentrifugiergeschwindigkeit V1 in Umdrehungen pro Minute (U/min),
- Bringen der Zentrifugiergeschwindigkeit auf einen Wert V2 in Umdrehungen pro Minute (U/min) größer als V1, und dabei Mischen einer bestimmten Menge des ätherischen Öls in Pulverform, das auf eine bestimmte Temperatur gebracht wird, mit der vorzentrifugierten bestimmten Menge von Algen, um ein Gemisch aus Algen und ätherischem Öl in einem ersten Zustand zu erhalten,
- Halten auf der Geschwindigkeit V2 der Zentrifugierung des Gemisches aus Algen und ätherischem Öl in dem ersten Zustand während einer Zeitdauer T1, um ein Gemisch aus Algen und ätherischem Öl in einem zweiten Zustand zu erhalten,
- Beaufschlagen des Gemisches aus Algen und ätherischem Öl in dem zweiten Zustand mit einem bestimmten Druck während einer Zeitdauer T2 in einer bestimmten Gasatmosphäre, um ein Gemisch aus Algen und ätherischem Öl in einem dritten Zustand zu erhalten;
- Zurückführen dieses Gemisches aus Algen und ätherischem Öl in einem dritten Zustand auf Atmosphärendruck und auf Umgebungstemperatur am Ende dieser Zeitdauer T2, um ein Gemisch aus Algen und ätherischem Öl in einem vierten Zustand zu erhalten,
- Beaufschlagen des Gemisches aus Algen und ätherischem Öl in dem vierten Zustand mit einer weiteren Zentrifugierung mit der Geschwindigkeit V3 in Umdrehungen pro Minute (U/min) und dabei Hinzufügen einer bestimmten Trockenextraktmenge von Pflanzen und von kolloidaler Kieselerde während einer Zeitdauer T3, um ein Endgemisch in einem fünften Zustand zu erhalten, und
- Ruhenlassen des Endgemisches in dem fünften Zustand während einer Zeitdauer T4, um ein Pulver zu erhalten, das geeignet ist, das Nahrungsergänzungsmittel auf Basis eines ätherischen Öls in Pulverform zu bilden.

2. Verfahren nach Anspruch 1, **dadurch gekennzeichnet, dass**:
- die Zentrifugiergeschwindigkeiten V1, V2 und V3 im Wesentlichen gleich 3000, 4500 bzw. 5000 U/min sind,
- die Zeitdauern T1, T2, T3 und T4 im Wesentlichen gleich einer Stunde, vier Stunden, fünfzehn Minuten und vierundzwanzig Stunden sind,
- der Wert der bestimmten Temperatur im Wesentlichen gleich 32 °C ist und
- der Wert des bestimmten Drucks im Wesentlichen gleich drei Bar ist.

3. Verfahren nach einem der Ansprüche 1 und 2, **dadurch gekennzeichnet, dass** die Gewichtsanteile der bestimmten Mengen von Algen, ätherischem Öl, kolloidaler Kieselerde und Pflanzen-Trockenextrakten im Wesentlichen gleich 65 %, 15 %, 1 % und 19 % sind.

4. Verfahren nach einem der vorhergehenden Ansprüche, **dadurch gekennzeichnet, dass** die Alge Lithothamnium ist.

5. Verfahren nach einem der vorhergehenden Ansprüche, **dadurch gekennzeichnet, dass** das ätherische Öl aus den folgenden ätherischen Ölen gewählt ist: echter Lavendel, Rosmarin Verbenon, grüner Anis, Teebaum, Zitrone, Cajeput, Zitroneneukalyptus, Waldkiefer, Lorbeer, ein Gemisch aus mindestens zwei dieser ätherischen Öle.

6. Verfahren nach einem der vorhergehenden Ansprüche, **dadurch gekennzeichnet, dass** der Pflanzen-Trockenextrakt aus den folgenden Pflanzen-Trockenextrakten gewählt ist: Melisse, Hopfenzapfen, grüner Oregano, Moosbeere, ein Gemisch aus mindestens zwei dieser Pflanzen.

7. Verfahren nach einem der vorhergehenden Ansprüche, **dadurch gekennzeichnet, dass** der Pflanzen-Trockenextrakt in der Größenordnung von 5/1 vorliegt, d. h., dass eine Gewichtseinheit Trockenextrakt im Mittel fünf Einheiten Pflanzenpulver entspricht.

8. Verfahren nach einem der vorhergehenden Ansprüche, **dadurch gekennzeichnet, dass** das Endgemisch in Kapseln gefüllt wird.

## Claims

1. A method of fabricating a food supplement based at least on an essential oil in powder form, the method being **characterized in that** it consists:
- in pre-centrifuging a certain quantity of alga at a centrifuging speed V1 in revolutions per minute (rpm);
- in raising the centrifuging speed to a value V2 in revolutions per minute (rpm) greater than V1 while mixing with said certain quantity of pre-centrifuged alga a certain quantity of essential oil in powder form raised to a determined temperature in order to obtain an alga-and-essential-oil mixture in a first state;
- in maintaining the centrifuging of the alga-and-essential-oil mixture in the first state at the speed V2 for a time duration T1 in order to obtain an alga-and-essential-oil mixture in a second state;
- in subjecting the alga-and-essential-oil mixture in said second state to a determined pressure for a time duration T2 under an atmosphere of a given gas in order to obtain an alga-and-essential-oil mixture in a third state;
- in returning, at the end of said time duration T2, the mixture of alga and essential oil in a third state to atmospheric pressure and to ambient temperature in order to obtain a mixture of alga and essential oil in a fourth state;
- in subjecting the alga-and-essential-oil mixture in said fourth state to further centrifuging at a speed V3 in revolutions per minute (rpm) while adding a certain quantity of dry extract of plants and of colloidal silica, for a time duration T3, in order to obtain a final mixture in a fifth state; and
- in leaving said final mixture in said fifth state to rest for a time duration T4 in order to obtain a powder suitable for constituting said food supplement based on essential oil in powder form.

2. A method according to claim 1, **characterized by** the fact that:
- the centrifuging speeds V1, V2, and V3 are respectively substantially equal to 3000 rpm, 4500 rpm, and 5000 rpm;
- the time durations T1, T2, T3, and T4 are substantially equal respectively to one hour, to four hours, to fifteen minutes, and to twenty-four hours;
- the value of the determined temperature is substantially equal to 32°C; and
- the value of the determined pressure is substantially equal to three bar.

3. A method according to claim 1 or claim 2, **characterized by** the fact that the proportions by weight of said certain quantities of alga, of essential oil, of colloidal silica, and of dry extracts of plants are respectively substantially equal to 65%, 15%, 1%, and 19%.

4. A method according to any preceding claim, **characterized by** the fact that said alga is lithothamnion.

5. A method according to any preceding claim, **characterized by** the fact that the essential oil is selected from the following essential oils: true lavender, verbenone rosemary, aniseed, tea tree, lemon, cajuput, eucalyptus citriodora, pine, bay, and a mixture of at least two of these essential oils.

6. A method according to any preceding claim, **characterized by** the fact that the dry extracts of plants is selected from the following dry extracts of plants: balm, hop cone, green oregano, cranberry, and a mixture of at least two of these plants.

7. A method according to any preceding claim, **characterized by** the fact that the dry extract of plants is of the order of 5/1, i.e. one unit by weight of dry extract is equivalent on average to five units of plant powder.

8. A method according to any preceding claim, **characterized by** the fact that said final mixture is put into capsules.
